# EUROPEAN PATENT APPLICATION

(11) **EP 1 600 181 A2**
(43) Date of publication of application: **30.11.2005**
(21) Application number: 05010497.5
(22) Date of filing: 13.05.2005
(51) Int. Cl.: A61L 31/12, A61L 31/16, A61F 2/06

(54) **Methods and compounds for treatment of aneurysmal tissue**

(30) Priority: 27.05.2004 US 574904 P; 22.09.2004 US 946939
(71) Applicant: Medtronic Vascular, Inc., Santa Rosa, California 95403 (US)
(72) Inventor: Tseng, David, Santa Rosa CA 95405 (US); Chu, Jack, Santa Rosa CA 95409 (US); Letort, Michel, 01280 Prevessins (FR); Raze, Brian, Ham Lake MN 55304 (US); Brin, David S., Topsfield MA 01983-1720 (US)
(74) Representative: Zimmermann & Partner

(57) **Abstract**

Methods and apparatus for minimizing the risks inherent in endovascular grafting for aneurysm repair are provided, including the implantation and time-controlled release of at least one bioactive agent at the aneurysmal site.

## Description

### FIELD OF THE INVENTION

The present invention relates to a compound to be delivered to an aneurismal site and a method for treating aneurismal tissue.

### BACKGROUND OF THE INVENTION

Aortic aneurysms pose a significant medical problem for the general population. Aneurysms within the aorta presently affect between two and seven percent of the general population and the rate of incidence appears to be increasing. Aneurysms are characterized by degeneration in the arterial wall in which the wall weakens and balloons outward by thinning. Until the affected artery is removed or bypassed, a patient with an aortic aneurysm must live with the threat of aortic aneurysm rupture and death.

One clinical approach for patients with an aortic aneurysm is aneurysm repair by endovascular grafting. Endovascular grafting involves the transluminal placement of a prosthetic arterial graft in the endoluminal position (within the lumen of the artery). To prevent rupture of the aneurysm, a stent graft of tubular construction is introduced into the aneurysmal blood vessel, typically from a remote location through a catheter introduced into a major blood vessel in the leg. The catheter/stent graft is then pushed through the blood vessel to the aneurysm location, and the stent graft is secured in a location within the blood vessel such that the stent graft spans the aneurysmal sac. The outer surface of the stent graft, at its ends, is sealed to the interior wall of the blood vessel at a location where the blood vessel wall has not suffered a loss of strength or resiliency, such that blood flowing through the vessel is diverted through the hollow interior of the stent graft, and thus is diverted from the blood vessel wall at the aneurysmal sac location. In this way, the risk of rupture of the blood vessel wall at the aneurysmal location is significantly reduced--if not eliminated--and blood can continue to flow through to the downstream blood vessels without interruption. The stent graft is sized such that upon placement into an aneurysmal blood vessel, the diameter of the stent graft slightly exceeds the existing diameter of the blood vessel at healthy blood vessel wall site on opposed ends of the aneurysm.

Despite the effectiveness of endovascular grafting, once the aneurysmal site is bypassed, the aneurysm remains. The aortic tissue can continue to degenerate such that the aneurysm increases in size due to thinning of the medial connective tissue architecture of the aorta and loss of elastin. Thus there is a desire in the art to achieve a greater success of aneurysm repair and healing. It is therefore an object of the present invention to overcome the problems associated with the prior art at least partially.

### SUMMARY OF THE INVENTION

This object is solved by a compound according to claim 1 and a method according to claim 22 and 24. Further aspects, features, advantages and details of the present invention are apparent from the dependent claims, the description, and the accompanying drawings.
Embodiments according to the present invention address the problem of aneurysm repair, particularly the problem of continued breakdown of aneurysmal tissue. A consequence of such continued breakdown is rupture of the aneurysm. Embodiments according to the present invention provide methods and compounds for supporting or bolstering the aneurysmal site following implantation of a graft, while supplying pharmaceutical agents to aid in stabilizing and healing the aneurysmal tissue.

Thus, in one embodiment according to the invention there is provided a compound comprising one or more biocompatible polymers and one or more bioactive agents, particularly those selected from tetracycline and the derivatives thereof, beta blockers such as propanolol, antiinflammatories, ACE inhibitors, COX-2 inhibitors and the like. Preferably, the one or more biocompatible polymers includes poly(butylmethacrylate) (PBMA), poly(ethylene-co-vinyl acetate) (PEVA), or polycaprolactone (PCL) or co-polymers thereof. More preferably in this embodiment, the polymer comprises PBMA having a molecular weight of between about 10,000 and 500,000, PEVA having a vinyl acetate concentration of less than about 50% or PLC having a molecular weight of about 5,000 to about 80,000.

In addition, in another embodiment of the present invention, there are provided methods for using these compounds, including, in one aspect, a method for treating aneurysmal tissue comprising implanting an endovascular stent graft in an individual with a delivery means tracked along side the endovascular graft where the distal end of the means reaches into the aneurysmal site; and delivering a compound comprising a biocompatible polymer and a bioagent to the aneurysmal site through the delivery means.

### BRIEF DESCRIPTION OF THE DRAWINGS

Figure 1 is a schematic view of a human aortal aneurysm.

Figure 2 is a partial sectional view of a descending aorta with a bifurcated stent graft and delivery means placed therein.

Figure 3 is a partial sectional view of a descending aorta with a bifurcated stent graft implanted therein and a compound according to the present invention delivered thereto.

### DETAILED DESCRIPTION

Embodiments according to the present invention encompass methods and compounds for stabilizing and treating an aneurysmal site subsequent to the implant of an endovascular stent/graft for aneurysm repair. Techniques include implanting an endovascular graft in an individual in a typical manner, where an appropriate delivery means, generally a catheter, has been tracked along side the graft with the distal end of the delivery means reaching into the aneurysmal sac. A compound comprising one or more biocompatible polymers and one or more bioactive agents, particularly those selected from tetracycline and the derivatives thereof, beta blockers such as propanolol, antiinflammatories, ACE inhibitors, COX-2 inhibitors and the like, are then delivered into the aneurysmal sac through the delivery catheter. Preferably, the one or more biocompatible polymers includes poly(butylmethacrylate) (PBMA), poly(ethylene-co-vinyl acetate) (PEVA), or polycaprolactone (PCL) or co-polymers thereof.

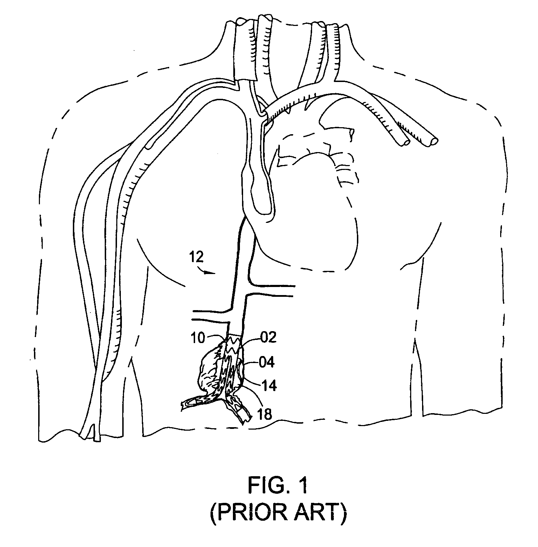

Referring initially to Figure 1, there is shown generally an aneurysmal blood vessel 02; in particular, there is an aneurysm of the aorta 12, such that the aorta or blood vessel wall 04 is weakened at an aneurysmal site 14 and the diameter of the aorta 12 at the aneurysmal site 14 is on the order of over 150% to 300% of the diameter of a healthy aorta 12. The aneurysmal site 14 forms an aneurysmal bulge or sac 18. If left untreated, the aneurysmal sac 18 may continue to deteriorate, weaken, increase in size, and eventually tear or burst. A stent graft 10 is shown.

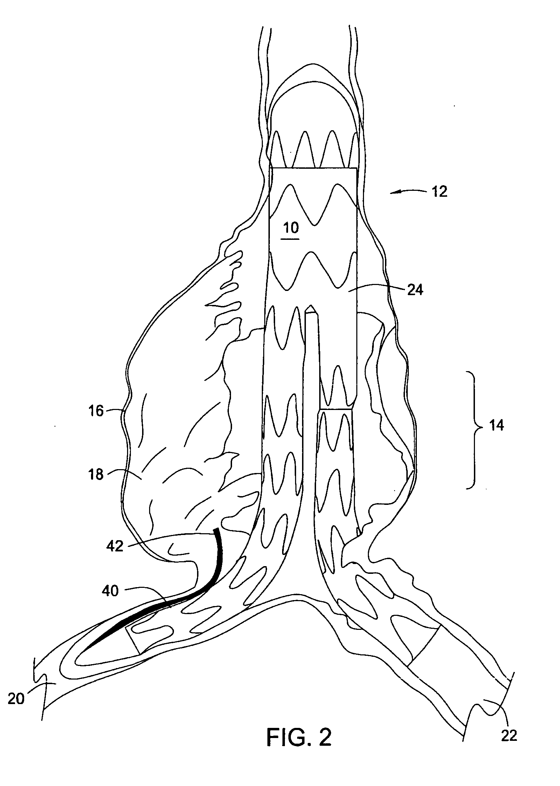

Figure 2 shows the transluminal placement of a prosthetic arterial stent graft 10, positioned in a blood vessel, in this embodiment, in, e.g., an abdominal aorta 12. The prosthetic arterial stent spans, within the aorta 12, an aneurysmal portion 14 of the aorta 12. The aneurysmal portion 14 is formed due to a bulging of the aorta wall 16, in a location where the strength and resiliency of the aorta wall 16 is weakened. As a result, an aneurysmal sac 18 is formed of distended vessel wall tissue. The stent graft 10 is positioned spanning the sac 18 providing both a secure passageway for blood flow through the aorta 12 and sealing of the aneurysmal portion 14 of the aorta 12 from additional blood flow from the aorta 12.

The placement of the stent graft 10 in the aorta 12 is a technique well known to those skilled in the art, and essentially includes opening a blood vessel in the leg or other remote location and inserting the stent graft 10 contained inside a delivery means, generally a catheter (not shown), into the blood vessel. The catheter/stent graft combination is tracked through the remote vessel until the stent graft 10 is deployed in a position that spans the aneurysmal portion 14 of aorta 12. In the embodiment shown in Figure 2, two delivery means are used. One or more delivery means are employed to deliver the stent graft into position at the aneurysmal site (not shown), and another delivery means is tracked along side one of the first delivery means (i.e., one of the delivery means used to deliver the stent graft into position). Once the stent graft is deployed, the other delivery means (shown here at 40) is in position to deliver a bioactive compound to the aneurysmal site through distal end 42.

The bifurcated stent graft 10 shown in Figure 2 has a pair of branched sections 20, 22 bifurcating from a trunk portion 24. This style of stent graft 10 is typically composed of two separate pieces, and is positioned first by inserting a catheter or other delivery means with the trunk portion 24 into place through an artery in one leg, providing a first branched section 20 to the aneurysmal location through the catheter and attaching it to the trunk portion at the aneurysmal site. Next, another catheter with the second branched section 22 is inserted into place through an artery in the other leg of the patient, positioning the second branched section 22 adjacent to the trunk portion 24 and connecting it thereto. The procedure and attachment mechanisms for assembling the stent graft 10 in place in this configuration are well known in the art, and are disclosed in, e.g., Lombardi, et al., US Pat No. 6,203,568. The delivery means used to deliver the bioagent to the aneurymsal site (shown at 40) can be tracked along side either the first or the second delivery means used to deliver the components of the stent graft.

A bioactive composition and related method for using the bioactive composition are provided to support an aneurysmal site following implantation of an endovascular graft in a manner that permits the release of a bioactive agent over time when the compound is delivered and implanted at the aneurysmal site in vivo.

The composition comprises a bioactive agent in combination with one or more polymers. The polymer components are adapted to be mixed to provide a compound that exhibits an optimal combination of physical characteristics (e.g., biocompatibility, biodegradability and bio-absorbability) and bioactive release characteristics. One preferred polymer composition comprises poly(butylmethacrylate) (PBMA), poly(ethylene-co-vinyl acetate) (PEVA), and/or polycaprolactone (PCL).

Other biodegradable compositions useful in embodiments according to the present invention include cellulose acetate, cellulose acetate proprionate, cellulose butyrate, cellulose proprionate, cellulose valerate, cumaroneindene polymer, dibutylaminohydroxypropyl ether, ethyl cellulose, ethylene-vinyl acetate copolymer, glycerol distearate, hydorxypropylmethyl cellulose phthalate, 2-methyl-5-vinylpyridine methylate-methacrylic acid copolymer, polyamino acids, polyanhydrides, polybutidiene, polyesters, aliphatic polyesters, polyhydroxybutyric acid, polymethyl methacrylate, polymethacrylic acid ester, polyolesters, polysaccharides (such as alginic acid, chitin, chitosan, chondroitin, dextrin or dextran), proteins (such as albumin, casein, collagen, gelatin, fibrin, fibrinogen, hemoglobin, or transfferrin), vinylchloride-propylene-vinylacetate copolymer, palmitic acid, stearic acid, behenic acid, aliphatic polyesters, hyaluronic acid, heparin, kearatin sulfate, starch, polystyrene, polyvinyl acetal diethylamino acetate, polyvinyl alcohol, polyvinyl butyral, polyvinyl formal, poly(D,L-lactide), poly(D,L-lactide-co-glycolide), poly(glycolide), poly(orthoglycolides), poly(orthoglycolide acrylates), poly(ortho acrylates), poly(hydroxybutyrate), poly(alkylcarbonate), poly(orthoesters), poly(hydroxyvaleric acid), polydioxanone, poly(malic acid), poly(tartronic acid), polyanhydrides, polyphosphazenes, and their copolymers or mixture.

The compositions and methods according to the present invention can be used to control the amount and rate of bioactive agent (e.g., drug) release at the aneurysmal site. One embodiment employs one or more hydrophobic polymers in combination with one or more bioactive agents-such as a pharmaceutical agent or other agents that aid in the treatment of aneurysmal tissue-such that the amount and rate of release of agent(s) can be controlled, e.g., by adjusting the relative types and/or concentrations of hydrophobic polymers in the mixture. For the combination of polymers taught herein, for instance, this approach permits the release rate to be adjusted and controlled by simply adjusting the relative concentrations of the co-polymers in the compound. The polymer mixture for use in an embodiment according to this invention is biocompatible such that it results in no induction of inflammation or irritation when implanted, degraded or absorbed. In addition, the polymer combination is useful under a broad spectrum of both absolute concentrations and relative concentrations of the co-polymers.

A class of polymers known as poly(butylmethacrylates) (PBMAs) provide an optimal combination of various structural/functional properties, including hydrophobicity, durability, bioactive agent release characteristics, biocompatability, molecular weight, and availability, and cost. Examples of PBMAs are those with molecular weights from 10,000 to 500,000 and are available commercially, e.g., from Aldrich with varying inherent viscosity, solubility, and form (e.g., as pellet or powder).

Another polymer component according to the present invention is poly(ethylene-co-vinyl acetate) (PEVA), which provides an optimal combination of properties similar to those of PBMA, particularly when used in admixture with PBMA. Suitable PEVAs are available commercially and have vinyl acetate concentrations of between about 0% and about 50%, in the form of beads, pellets, granules, etc. (commercially available are 12%, 14%, 18%, 25%, 33%). PEVA co-polymers with a lower percentage of vinyl acetate become increasingly insoluble in typical solvents, whereas those with higher percentage of vinyl acetate become decreasingly durable.

A third class of polymers, polycaprolactones (PCLs), also provide an optimal combination of various structural/functional properties, including hydrophobicity, durability, bioactive agent release characteristics, biocompatability, molecular weight, and availability, and cost. Examples of PCLs are those with molecular weights from 5000 to 80,000. Such PCL polymers are available commercially, e.g., from Birmingham Polymers, Inc. with varying inherent viscosity, solubility, and form (e.g., as pellet or powder).

One polymer mixture for use in an embodiment according to this invention is a mixture of PBMA and PEVA. Such a mixture of polymers has proven useful with absolute polymer concentrations (i.e., the total combined concentrations of the polymers in the composition), of between about 40% and about 99% (by weight). It has furthermore proven effective with individual polymer concentrations in the coating solution of between about 5 and about 50 weight percent. In one embodiment the polymer mixture includes PBMA with a molecular weight of from 10,000 to 500,000; a PEVA copolymer with a molecular weight of from 10,000 to 500,000 and a vinyl acetate content of from 0 to 50 weight percent. In another embodiment, the a polymer mixture includes PBMA with a molecular weight of from 10,000 to 33,000; a PEVA copolymer with a molecular weight of from 10,000 to 40,000 and a vinyl acetate content of from 5 to 30 weight percent. The concentration of the bioactive agent or agents dissolved or suspended in the compound can range from 1 to 50 percent, by weight, based on the percent weight of the combined co-polymers.

As stated previously, endovascular grafts have proven successful in patients with aortic aneurysms; however, despite such grafts, the aneurysmal tissue may continue to degenerate due to thinning of the medial connective tissue architecture of the aorta and a concommitent loss of elastin. There appears to be no turnover of connective tissue and there is little connective tissue-degrading enzymatic activity in a healthy adult aorta; however, there is evidence that connective tissue degrading elastolytic activity can increase following insult or trauma to a blood vessel, and that this phenomenon is associated with dilatation of the vessel. Thus, among other advantages, the invention provides a method for protecting elastic fibers in the medial lamellae of blood vessels from abnormal degradation which can otherwise lead to continued dilatation and/or further degeneration of the aortic tissue. Accordingly, one aspect according to the present invention involves selectively inhibiting elastolytic activity at the aneurysmal site. Elastolytic activity includes enzymatic-associated structural deterioration of arterial elastin and associated elements such as extracellular matrix components. Thus, this aspect of the invention encompasses the selective inhibition of such abnormal or elevated elastolytic activity in the vascular tissue by providing compounds and methods for contacting vascular tissue with an inhibitor of elastolytic activity in an amount (an anti-elastolytic amount) sufficient to selectively inhibit elastolytic activity in the tissue.

It has been shown that medial thinning and loss of elastin in the aorta may be due, in part, to the effects of matrix metalloproteinases (MMPs). MMPs are a group of proteolytic enzymes associated with extracellular matrix. MMPs are known to degrade one or more connective tissue elements and have been implicated in clearing a path through the extracellular matrix for cell migration. MMPs have been shown to have important roles in embryogenesis, tumor invasion, arthritis and atherosclerosis. For these reasons, there is interest in matrix metalloproteinase inhibitors (MMPls) or tissue inhibitors of metalloproteinases (TIMPs). MMPs include MMP1, interstitial collagenase; MMP2, gelatinase A; MMP3, stromelysin-1; MMP8, neutrophil collagenase; MMP9; MMP10, stromelysin-2; and MMP 12, metalloelastase.

The inhibition of elastolytic activity is preferably selectively directed against proteolytic activity associated with tissue matrix metalloproteases. Among other anti-metalloprotease effects, the invention is effective to accomplish the selective inhibition of gelatinase, i.e., MMP-2 and MMP-9. Thus, in general, inhibitors of matrix metalloproteases and more specifically inhibitors of gelatinase, are to be used as bioactive agents in the compounds according to the present invention.

In one embodiment, a method according to the invention involves administration of an anti-aneurysmal amount of a bioactive reagent/polymer compound to an individual in need of anti-aneurysmal therapy. An anti-aneurysmal amount of a bioactive agent in this context is an amount that inhibits the progression or induces the regression of an established (pre-existing) aneurysm. Suitable inhibitors include, for example, endogenous inhibitors, such as tissue inhibitors of MMPs (TIMPs) and macroglobulins, and synthetic inhibitors, such as chelating agents (e.g., EDTA and 1,10-phenanthroline), peptides, antibodies, and antibiotics such as tetracycline and its derivatives.

The maximal dosage of the bioactive agent for a mammal is the highest dosage that effectively inhibits elastolytic anti-aneurysmal activity, but does not cause undesirable or intolerable side effects. In any event, the practitioner is guided by skill and knowledge in the field, and the present invention includes without limitation dosages that are effective to achieve the described phenomena.

A particular embodiment according to the present invention employs any suitable tetracycline or tetracycline-derivative compound, preferably doxycycline hydrate, having an anti-aneurysmal effect. In using a tetracycline compound as the bioactive agent administered according to the invention, the observed anti-aneurysmal effect appears to be unrelated to, and independent of, any antimicrobial activity such a compound might have. Accordingly, the tetracycline may be an antimicrobial tetracycline compound, or it may be a tetracycline analogue having little or no significant antimicrobial activity.

Preferred antimicrobial tetracycline compounds (anti-aneurysmal agents) include, for example, tetracycline per se, as well as derivatives thereof. Preferred derivatives include, for example, doxycycline aureomycin and chloromycin. If a tetracycline analogue having little or no antimicrobial activity is to be employed, it is preferred that the compound lack the dimethylamino group at position 4 of the ring structure of tetracycline. Such chemically-modified tetracyclines include, for example, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, and 6α-deoxy-5-hydroxy-4-dedimethylaminotetracycline. Also, tetracyclines modified at the 2-carbon position to produce a nitrile, e.g., tetracyclinonitrile, are useful as non-antibacterial, anti-metalloproteinase agents. Further examples of tetracyclines modified for reduced antimicrobial activity include 6-α-benzylthiomethylenetetracycline, the mono-N-alkylated amide of tetracycline, 6-fluoro-6-demethyltetracycline and 11-α-chlorotetracycline.

Another class of bioactive agent that finds utility in embodiments according to the present invention for inhibiting the progression or inducing the regression of a pre-existing aneurysm is beta blockers or beta adrenergic blocking agents. Beta blockers are bioactive agents that reduce the symptoms associated with hypertension, cardiac arrhythmias, angina pectoris, migraine headaches, and other disorders related to the sympathetic nervous system. Beta blockers also are often administered after heart attacks to stabilize the heartbeat. Within the sympathetic nervous system, beta-adrenergic receptors are located mainly in the heart, lungs, kidneys and blood vessels. Beta blockers compete with nerve-stimulation by the hormone epinephrine for these receptor sites and thus interfere with the action of epinephrine, lowering blood pressure and heart rate, stopping arrhythmias, and preventing migraine headaches. Because it is also epinephrine that prepares the body for "fight or flight," in stressful or fearful situations, beta-blockers are sometimes used as anti-anxiety drugs, especially for stage fright and the like. There are two main beta receptors, beta 1 and beta 2. Some beta blockers are selective, such that they selectively block beta 1 receptors. Beta 1 receptors are responsible for the heart rate and strength of the heartbeat. Nonselective beta blockers block both beta 1 and beta 2 receptors. Beta 2 receptors are responsible for the function of smooth muscle.

Beta blockers that may be used in the compounds and methods according to the present invention include acebutolol, atenolol, betaxolol, bisoprolol, carteolol, carvedilol, esmolol, labetolol, metoprolol, nadolol, penbutolol, pindolol, propranolol, and timolol, as well as other beta blockers known in the art.

Other therapeutic agents useful in embodiments according to the present invention include, for example, cyclooxygenase-2 (COX-2) inhibitors, angiotensin-converting enzyme (ACE) inhibitors, glucocorticoids, nitric acid synthase (NOS) inhibitors, other anti-inflammatories, antioxidants and cellular adhesion molecules (CAMs). COX-2 inhibitors include Celecoxib, Rofecoxib, Valdecoxib, Etoricoxib, and Parecoxib, all of which are available in pharmacological preparations. Additionally, COX-2 inhibition has been demonstrated from herbs such as green tea, ginger, turmeric, chamomile, Chinese gold-thread, barberry, baikal skullcap, Japanese knotweed, rosemary, hops, feverfew, and oregano; and from other agents such as piroxican, mefenamic acid, meloxican, nimesulide, diclofenac, MF-tricyclide, raldecoxide, nambumetone, naproxen, herbimycin-A, and diaryl hydroxyfuranones. NSAIDs that may be used in embodiments according to the present invention include ketoralac tromethamine (Toradol), indomethacin, ketorolac, ibuprofen and aspirin, among others. Additionally, steroidal based anti-inflammatories, such as methylprednisolone, dexamethasone or sulfasalazine may be provided to reduce the inflammation at the aneurysmal site. Other suitable anti-inflammatory agents include cyclosporine A and azathioprine. Another type of suitable therapeutic agent are the anti-oxidants, such as curcumin, vitamins, and vitamin constituents, such as (?)-tocopherol and (?)-carotene. Yet other therapeutic agents useful in embodiments according to the present invention are angiotensin-converting enzyme (ACE) inhibitors that suppress the development of elastase-induced vessel damage. Such ACE inhibitors known in the art are captopril, enalapril, losartan and lisinopril and the active forms of several ACE inhibitor prodrugs on the market. Other agents such as the NOS inhibitor aminoguanidine are also useful in embodiments according to the present invention.

The polymer/bioactive agent compound can be formulated as a gel, a paste, in pellets, or in any other effective configuration or formulation that is amenable to delivery from the delivery means chosen, such as a catheter.

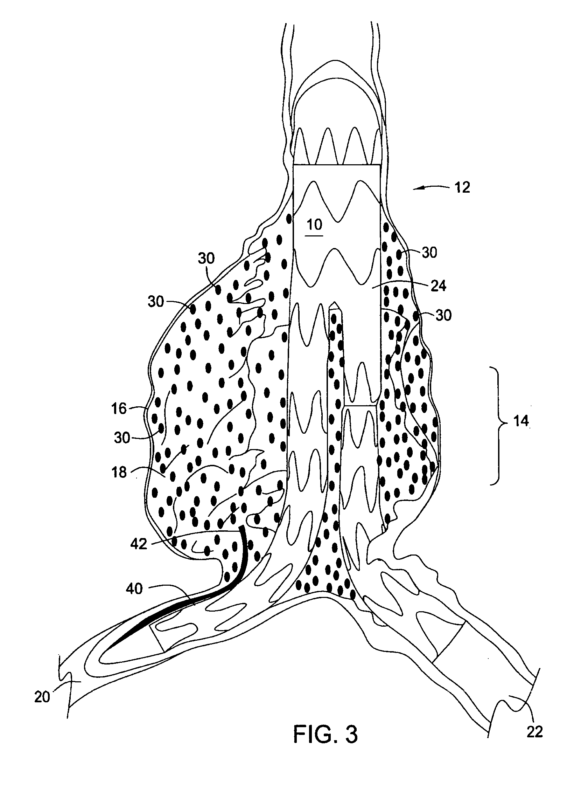

Figure 3 is similar to Figure 2 and shows the transluminal placement of a prosthetic arterial stent graft 10, positioned in a blood vessel, in this embodiment, in, e.g., an abdominal aorta 12. The prosthetic arterial stent spans, within the aorta 12, an aneurysmal portion 14 of the aorta 12. As in Figure 2, an aneurysmal portion 14 is formed due to a bulging of the aorta wall 16, in a location where the strength and resiliency or the aorta wall 16 is weakened, and an aneurysmal sac 18 is formed of distended vessel wall tissue. The stent graft 10 is positioned spanning the sac 18 providing both a secure passageway for blood flow through the aorta 12 and sealing of the aneurysmal portion 14 of the aorta 12 from additional blood flow from the aorta 12. Also shown surrounding the stent graft at the aneurysmal site are pellets of a bioactive compound according to the present invention (30), which have been delivered into the aneurismal sac 18 through the distal end 42 of the delivery means 40.

While the present invention has been described with reference to specific embodiments, it should be understood by those skilled in the art that various changes may be made and equivalents may be substituted without departing from the true spirit and scope of the invention. In addition, many modifications may be made to adapt a particular situation, material or process to the objective, spirit and scope of the present invention. All such modifications are intended to be within the scope of the invention.

All references cited herein are to aid in the understanding of the invention, and are incorporated in their entireties for all purposes.

## Claims

1. A compound to be delivered to an aneurysmal site for selectively inhibiting further degradation of vascular tissue comprising a biocompatible polymer and a bioagent formulated to be implanted in the aneurysmal site.

2. The compound of claim 1, wherein the polymer comprises PMBA, PEVA, polymer blend or copolymers thereof.

3. The compound of claim 2, wherein the polymer is a polymer of PBMA having a molecular weight of between about 10,000 and 500,000, PEVA having a vinyl acetate concentration of less than about 50% or polymer blends thereof.

4. The compound of claim 3, wherein the PBMA has a molecular weight of about 10,000 to about 33,000, the PEVA has a molecular weight of about 10,000 to about 40,000 and has a vinyl acetate content of about 5% to 30% by weight.

5. The compound of any of the preceding claims, wherein the polymer comprises PCL, or copolymers or polymer blends thereof.

6. The compound of claim 5, PLC having a molecular weight of about 5,000 to about 80,000 or polymer blends thereof.

7. The compound of claim 1, wherein the polymer comprises poly(butylmethacrylate) (PBMA), poly(ethylene-co-vinyl acetate) (PEVA), polycaprolactone (PCL), cellulose acetate, cellulose acetate proprionate, cellulose butyrate, cellulose proprionate, cellulose valerate, cumaroneindene polymer, dibutylaminohydroxypropyl ether, ethyl cellulose, ethylene-vinyl acetate copolymer, glycerol distearate, hydorxypropylmethyl cellulose phthalate, a 2-methyl-5-vinylpyridine methylate-methacrylic acid copolymer, a polyamino acid, a polyanhydride, polybutidiene, a polyester, an aliphatic polyester, polyhydroxybutyric acid, polymethyl methacrylate, polymethacrylic acid ester, a polyolester, a polysaccharide, a protein), vinylchloride-propylene-vinylacetate copolymer, palmitic acid, stearic acid, behenic acid, an aliphatic polyester, hyaluronic acid, heparin, kearatin sulfate, starch, polystyrene, polyvinyl acetal diethylamino acetate, polyvinyl alcohol, polyvinyl butyral, polyvinyl formal, poly(D,L-lactide), poly(D,L-lactide-co-glycolide), poly(glycolide), a poly(orthoglycolide), a poly(orthoglycolide acrylate), a poly(ortho acrylate), a poly(hydroxybutyrate), a poly(alkylcarbonate), a poly(orthoester), poly(hydroxyvaleric acid), polydioxanone, poly(malic acid), poly(tartronic acid), a polyanhydride, a polyphosphazene, or a copolymer thereof.

8. The compound of claim 7, wherein the polymer comprises blend of above polymers.

9. The compound of any of the preceding claims, wherein the polymer comprises about 50% to about 99% by weight of the compound.

10. The compound of claim 6, wherein the compound is a polymer blend of at least two of PBMA, PEVA or PCL, and each polymer blend comprises between about 2% and about 50% by weight of the compound.

11. The compound of any of the preceding claims, wherein the bioagent comprises about 1% to about 50% by weight of the compound.

12. The compound of any of the preceding claims, wherein the bioagent inhibits elastolytic activity at the aneurysmal site.

13. The compound of any of the preceding claims, wherein the bioagent is a tissue inhibitor of MMPs, a macroglobulin, a chelating agent, a peptide, an antibody, an anti-inflammatory, an ACE inhibitor, an NSAID, a COX-2 inhibitor, an antibiotic, MMP inhibitors or any combination of these bioagents

14. The compound of claim 13, wherein the bioagent is EDTA or 1, 10-phenanthroline.

15. The compound of claim 13 or 14, wherein the bioagent is tetracycline or a derivative of tetracycline.

16. The compound of claim 15, wherein the derivative of tetracycline is doxycycline aureomycin, chloromycin, 4-dedimethylaminotetracycline, 4-dedimethylamino-5-oxytetracycline, 4-dedimethylamino-7-chlorotetracycline, 4-hydroxy-4-dedimethylaminotetracycline, 5 a, 6-anhydro-4-hydroxy-4-dedimethylaminotetracycline, 6-demethyl-6-deoxy-4-dedimethylaminotetracycline, 4-dedimethylamino-12a-deoxytetracycline, 6-α-deoxy-5-hydroxy-4-dedimethylaminotetracycline, tetracyclinonitrile, 6-α-benzylthiomethylenetetracycline, 6-fluoro-6-demethyltetracycline, or 11-α-chlorotetracycline.

17. The compound of any of claims 13 to 16, wherein the bioagent is a beta blocker.

18. The compound of claim 17, wherein the beta blocker is selected from acebutolol, atenolol, betaxolol, bisoprolol, carteolol, carvedilol, esmolol, labetolol, metoprolol, nadolol, penbutolol, pindolol, propranolol, or timolol.

19. The compound of any of claims 13 to 18, wherein the bioagent is anti inflammatory agent selected from Indomethacin, Diclofenac, mefanamic acid, Dexamethasone, Methylprednisolone, Piroxicam, or Naproxen.

20. The compound of any of claims 13 to 19, wherein the bioagent is a COX-2 inhibitor selected from Rofecoxib, Celecoxib, or Valdecoxib.

21. The compound of any of claims 13 to 20, wherein the bioagent is an ACE inhibitor selected from Caplopril, Lisinopril, Enalapril, or Losartan.

22. A method for treating aneurysmal tissue in an individual comprising the step of implanting the compound of claim 1 at an aneurysmal site.

23. The method of claim 22, further comprising:
implanting an endovascular stent graft in the individual where a delivery means has been tracked along side the endovascular graft with a distal end of the means reaching to the aneurysmal site; and
delivering the compound to the aneurysmal site through the delivery means.

24. A method for treating aneurismal tissue in an individual comprising:
implanting an endovascular stent graft in the individual where a delivery means has been tracked along side the endovascular graft with a distal end of the means reaching to the aneurysmal site; and
delivering a compound comprising a biocompatible polymer and a bioagent to the aneurysmal site through the delivery means.
